(19) **Europäisches Patentamt European Patent Office Office européen des brevets**

(11) **EP 4 253 515 B1**

(12) **EUROPEAN PATENT SPECIFICATION**

(45) Date of publication and mention
of the grant of the patent:
**22.07.2026 Bulletin 2026/30**

(51) International Patent Classification (IPC):
*C12M 1/36* (2006.01)     *B01F 33/40* (2022.01)
*C12M 1/00* (2006.01)

(21) Application number: **22305436.2**

(22) Date of filing: **01.04.2022**

(52) Cooperative Patent Classification (CPC):
**B01F 33/403; B01F 33/406; C12M 29/08;
C12M 41/48**

(54) **OPTIMIZED HAIRY ROOTS BIOREACTOR**

OPTIMIERTER HAARWURZEL-BIOREAKTOR

BIORÉACTEUR DE RACINES CHEVELUES OPTIMISÉ

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(43) Date of publication of application:
**04.10.2023 Bulletin 2023/40**

(73) Proprietor: **Samabriva**
**80000 Amiens (FR)**

(72) Inventors:
 • **CARDON, Florian**
**80000 Amiens (FR)**

 • **LEMASSON, Camille**
**80000 Amiens (FR)**
 • **GUILLET, Marina**
**80000 Amiens (FR)**

(74) Representative: **Icosa**
**83 avenue Denfert-Rochereau**
**75014 Paris (FR)**

(56) References cited:
**WO-A2-2008/135991**    **GB-A- 2 202 549**
**US-A- 4 649 117**    **US-A1- 2007 037 279**

## Description

## FIELD OF INVENTION

The present invention relates to growing biomass, in particular with the aim of producing molecules of interest (like for example recombinant proteins and/or secondary metabolites), in an optimized bioreactor.

## BACKGROUND OF INVENTION

Hairy roots from plants have been widely studied and used for the production of specialized/secondary metabolites of industrial and pharmaceutical interest. Since the 1990s, the production of recombinant proteins has been considered as another promising application of hairy root cultures. This system presents numerous similarities with the one used to produce recombinant proteins from mammalian cell lines (such as, *e.g.,* Chinese hamster ovary (CHO) cells, human cell lines, bacteria), among which the fact that the whole process is maintained under sterile conditions in a confined environment.

This plant-based technology offers relevant and advantageous differences compared to the mammalian expression systems. The selected hairy root clones are easily grown in tailor-made optimal conditions in a simple culture medium which has a better safety profile than culture media containing human- or animal-derived constituents. This process allows the large-scale production of compounds (often referred to as molecules of interest) such as recombinant proteins or secondary metabolites in an axenic environment in bioreactors. Moreover, contrary to the common mammalian cells-based expression systems, the hairy-root expression system allows a straightforward recovery of the molecules of interest that does not require an additional extraction phase.

However, as in every production method, it is important to obtain the highest possible amount of molecules of interest (like proteins or metabolites) in a same bioreactor in order to preserve an optimal reproducibility and decrease the number of
bioreactors used in parallel in an attempt to reduce the carbon footprint. Document WO2008/135991 A2 discloses a bioreactor with a plurality of gas inlets. Document US2007/037279 discloses a bioreactor providing generation of single large gas bubbles.

## SUMMARY

This invention thus relates to a bioreactor as defined by claim 1.

Thus, this solution achieves the above objective. In particular, it makes it possible to obtain a significant improvement in the productivity of molecules of interest while maintaining a high biomass growth by using bubbles ranging from 5 mm to 17 mm of diameter.

The bioreactor according to the invention may include one or more of the following characteristics, taken in isolation from one another or in combination with one another:

- the sparger may be arranged and secured in the center of the closed extremity, centered with regard to the revolution axis X,
- the sparger may comprise at least one radial aperture located along a revolution axis A of the sparger, said aperture being designed to generate a column of bubbles,
- the sparger may comprise two alignments of at least one radial aperture located along a revolution axis A of the sparger, the two alignments being on opposite sides of the sparger, said apertures being designed to generate at least one column of bubbles,
- the bioreactor may be configured to contain at least 100 liters of liquid,
- the bioreactor may be configured to contain at least 350 liters of liquid,
- the bioreactor may be configured to contain at least 1000 liters of liquid,
- the internal tank may be disposable,
- the bioreactor may further comprise a control system,
- the closed extremity may display a conical shape centered around the revolution axis,
- the sparger may be designed to cooperate with the conical shape of the closed extremity,
- the bioreactor may be an air-lift system in which the liquid is solely put in motion by the generated air bubbles.

A further object of the present invention is a system for the production of biomass, said system comprising a bioreactor as defined bone of the claims 1-12 and a gas injection device, the gas injection device being put in fluidic communication with the sparger.

A third object of the present invention is a method for growing biomass in a culture medium, said culture medium being put in motion by a bioreactor as defined by one of the claims 1-12, said method comprising the following steps:

- filling the internal tank with culture medium,
- inserting the biomass aimed at being grown in the culture medium,
- feeding the sparger with gas in order to generate the bubble column aimed at putting the culture medium into motion.

## BRIEF DESCRIPTION OF THE DRAWINGS

The invention will be better understood, and other aims, details, characteristics and advantages thereof will emerge more clearly on reading the detailed explanatory description which follows, including embodiments of the invention given by way of illustration and purely illustrative and non-limiting examples, with reference to the accompanying drawings:

- **Figure 1** is a schematic view of the bioreactor according to the invention,
- **Figure 2** is a perspective view of the bioreactor according to the invention filled with liquid,
- **Figure 3** is a schematic view of the internal tank of the bioreactor according to the present invention filled with gas and liquid,
- **Figure 4** is a schematic longitudinal section of a first embodiment of the internal tank of the bioreactor according to the present invention,
- **Figure 5** is a schematic longitudinal section of a second embodiment of the internal tank of the bioreactor according to the present invention,
- **Figure 6** is a transparent perspective view from a sparger according to the present invention,
- **Figure 7** is a view from above from the sparger according to figure 6,
- **Figures 8a and 8b** are longitudinal section views of the sparger according to figure 6,
- **Figure 9** are charts comparing bubble surfaces in function of water heights using different sorts of spargers,
- **Figure 10** are charts comparing biomass concentration and protein productivity using respectively a wave bioreactor and a large bubble sparger according to the present invention,
- **Figure 11** are charts comparing biomass concentration and protein productivity using respectively a microbubble sparger and a large bubble sparger according to the present invention.

**DETAILED DESCRIPTION**

The invention thus relates to a bioreactor 10 for the production of biomass, such as hairy roots, in particular with the aim of producing molecules of interest. The bioreactor is a column like reactor extending along a longitudinal axis X with a bottom extremity 10A and an upper extremity 10B.

In the present invention, the terminology "biomass" refers to any living organisms that may be cultured (or grown) *in vitro.* In some embodiments, the biomass refers to a plant (*i.e.,* plant biomass), in particular to plant roots (*i.e.,* root biomass). Example of plant roots that may be grown in the bioreactor include hairy roots, rhizocals, and adventitious roots. Thus, in some embodiments, the biomass is adventitious roots, hairy roots, rhizocals, or any combination thereof. In some embodiments, the biomass is hairy roots (*i.e.,* hairy root biomass).

The terminology "adventitious roots" refers to root emergences which appear during the physiological development of the plant and hence occurs naturally. The terminology "hairy roots" refers to root emergences which appear after the infection of a plant by *Rhizobium rhizogenes* (previously referred to as *Agrobacterium rhizogenes*) bacteria or by *Rhizobium radiobacter* (also known as *Agrobacterium Tumefaciens*) bacteria harboring *rol* genes for example. The terminology "rhizocal" or "rhizocallus" refers to a conic-shaped structure connected to the roots (in particular to the hairy roots), also termed lateral root emergence, which develops alongside of the roots in a solidarized way. In practice, rhizocals (or "rhizocalli") may be induced in a culture of roots by the addition of auxin in the culture medium.

The terminology "molecule of interest" refers to molecules (or compounds), such as recombinant proteins and/or secondary metabolites, which may be produced (and likely secreted) by the biomass, in particular by roots such as hairy roots.

The bioreactor 10, more precisely the internal tank 14 of the bioreactor 10, is configured to contain at least 15 liters of a liquid. In the present invention, the terminology "a liquid" or "liquid" refers to at least one liquid. In the other words, in the present invention, the terminology "a liquid" or "liquid" may include one or several liquids. In some embodiment, the liquid is a culture medium.

Roots, and in particular hairy roots are commonly cultivated (or grown) on solid medium or in liquid medium. Hairy roots may be cultivated in a standard culture medium (such as Gamborg B5, MS, etc.) or using an optimized culture medium where each nutrient concentration is specifically defined to allow better growth and/or molecule productivity. In a preferred embodiment, the internal tank 14 of the bioreactor 10 is configured to contain a volume ranging from 200 to 350 L of liquid. In some embodiments, the internal tank 14 of the bioreactor 10 can contain up to 1000 L of liquid, and even up to 5000 L. The internal tank 14 is classically put together by welding several pieces together.

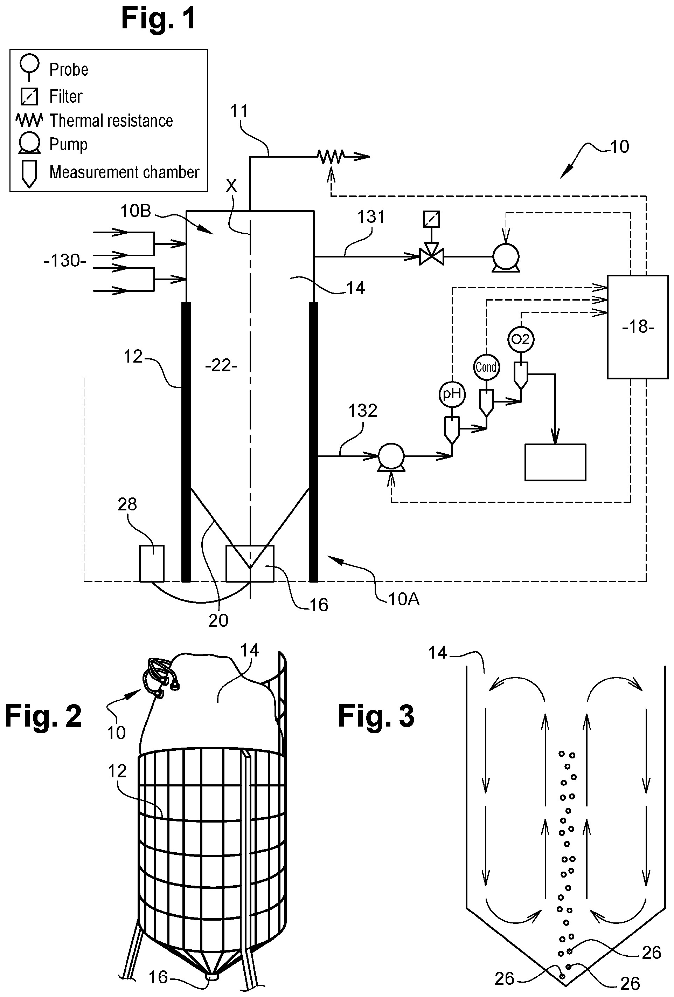

In these regards, as can be seen on the embodiment depicted on figure 1, the bioreactor 10 comprises:

- an external support structure 12 displaying a general annular shape extending along a revolution axis X, also being the longitudinal axis X,
- a generally cylindrical shaped internal tank 14, being configured to be secured inside and supported by the external support structure 12,
- a sparger 16 located at the closed extremity 20 of the internal tank 14,
- a control system 18 comprising hardware and software.

The bioreactor 10 according to the present invention might be part of a system for the production of biomass, said system comprising a bioreactor 10 and a gas injection device put in fluidic communication with the sparger 16.

The external support structure 12 is designed to hold the internal tank 14 as a sheath, as can be seen on figure 2. In some embodiment, as can be seen on figure 1, the external support structure 12 can be made of a stainless-steel mesh. The external support structure 12 may thus withstand the pressure applied by the liquid inside the internal tank 14 and avoid that this pressure is withstood solely by the plastic and welding sites of the internal tank 14. This allows to avoid any leaks at the different welding sites.

As can also be seen on figure 2, the internal tank 14 is made of flexible material, as for example, a polypropylene/polyamide double-layer plastic film, displaying a thickness of 115 $\mu$m. In any case, the material should be a gas and liquid barrier plastic film.

In a preferred embodiment, the internal tank 14 is disposable. This makes it easier to grow the biomass in sterile conditions.

More precisely, the internal tank 14 is made of five strips welded together. This makes it possible, once the internal tank 14 is filled with liquid, to form a reservoir much closer to a cylinder than if the internal tank 14 had been made from one or two strips. This allows the pressure forces exerted by the liquid filling the internal tank 14 to be distributed more evenly over the entire internal surface of the internal tank and along the different welding sites.

The internal tank 14 displays at least one closed extremity 20. In some embodiments, said closed extremity 20 can display a conical shape, the cone being centered around the revolution axis X. The conical shape of the closed extremity 20 of the internal tank 14 of the bioreactor 10 makes it possible both to reduce dead volumes and also to facilitate recirculation of the liquid. This closed extremity 20 is configured to be the bottom extremity 10A of the bioreactor 10. In some embodiments, in which the biomass has to been grown in a sterile environment, the upper extremity of the internal tank 14 (being the upper extremity 10B of the bioreactor 10) is also closed and comprises an air outlet 11. Said upper extremity may further comprise several inlet conducts 130 to add liquid and nutrients, one first outlet conduct 131 to collect sample and one second outlet conduct 132 to collect trash.

Depending on the embodiments, the internal tank 14 presents a height ranging from 1000 to 5000mm and a width ranging from 300 to 1000mm.

The internal volume 22 of the internal tank 14 is free of any internal structure, in particular any internal support structure. This means that the internal volume 22 of the internal tank 14 is one single continuous space: no internal support structure like a transverse or crossing conduct, an internal strand or an internal wall is to be found inside the internal tank 14 in order to separate this unique internal volume 22 into several internal spaces.

In some embodiments, the internal tank 14 comprises at least one loop 24 secured to its wall. The at least one loop 24 is configured to hold at least one fluidic conduct and to maintain said fluidic conduct in contact with the wall. The fluidic conduct might be used to transport some water or fresh culture medium. In some embodiments, the loop 24 is secured on the external face of the wall of the internal tank 14 and in some alternative embodiments, the loop 24 is secured on the internal face of the wall of the internal tank 14. However, even if the loop 24 is secured on the internal face of the wall, said loop and the associated fluidic conduct being maintained in contact with the wall, they do not separate the unique internal volume 22 into several internal spaces. Neither the loop 24 nor the fluidic conducts can be considered as internal support structures.

The design of the internal tank 14 of the bioreactor 10 includes different factors among which the geometric ratio ($r_G$), which is defined, in the present application, as being the ratio between the diameter d and the useful height H (H being the height of the liquid column inside the internal tank 14) of the internal tank 14:

$$r_G(-) = \frac{d\,(m)}{H\,(m)}$$

This ratio defines the size of the internal tank 14 and thus the space available for the biomass (for example hairy roots) to grow. Different ratios can be used regarding the present invention depending on the volume of the internal tank 14. For a 25L scale, $r_G$ ranges between 0.1 and 0.2, for a 200L scale, $r_G$ ranges between 0.3 and 0.5 and for a 350L scale, $r_G$ ranges between 0.4 and 0.6.

The sparger 16 and the internal tank 14 are arranged in a way that the generated bubbles 26 form a single bubble column once the internal tank 14 is filled with liquid and the sparger 16 is fed with gas. Said column is one single bubble column. The bubble 26 column enables the liquid to be put in motion.

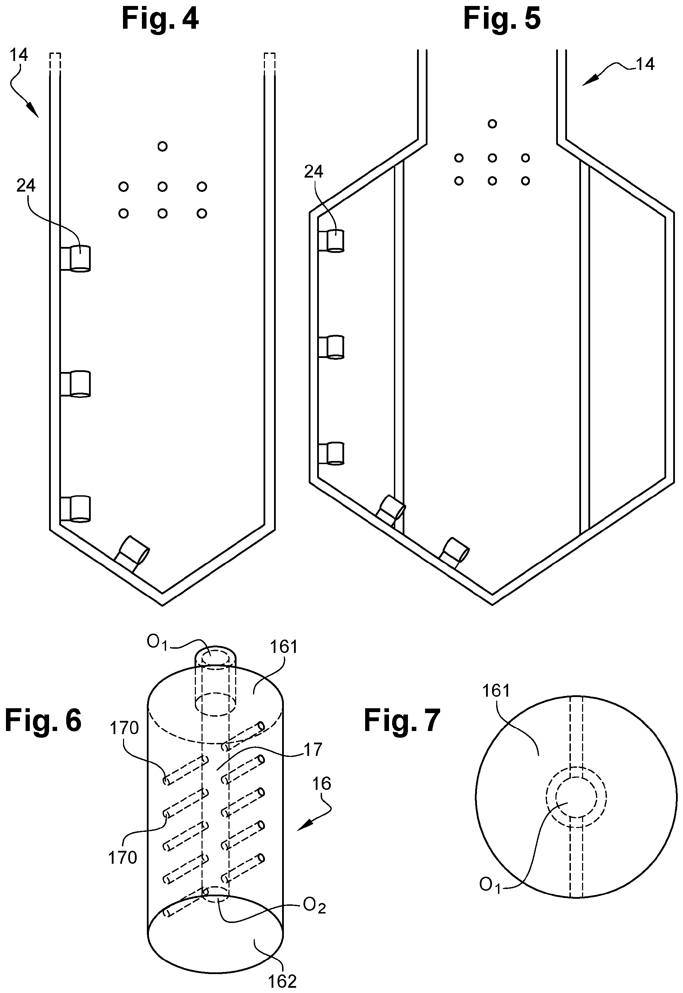

As can be seen on figure 6, the sparger 16 preferably displays a general cylindrical shape extending along a revolution axis A with a diameter ranging from 10 to 20mm and a length ranging from 30mm to 100mm. When the sparger 16 is inserted in the internal tank 14, the revolution axis A of the sparger 16 aligns with the revolution axis X of the internal tank 14. As can be grabbed on figures 6, 7 and 8a, the sparger 16 comprises an upstream extremity 161 and a downstream extremity 162. The upstream extremity 161 comprises an opening O which enables the sparger to be fed with gas. The opening O extends, along the revolution axis A, outwards the upstream extremity 161. This shape enables a better gas distribution inside the sparger 16 and avoids any dead volumes.

The walls of the sparger can be made of inox, metal or plastic, for example.

An internal conduct 17 presenting a diameter ranging from 2 to 5mm extends downstream from the opening O and enables gas to enter the sparger 16 through the opening O. The sparger 16 further comprises, on its external wall, at least one radial aperture 170 put radially in fluidic communication with the internal conduct 17. In some embodiments, the sparger 16 comprises several radial apertures 170. The opening O is thus put in fluidic communication with each radial aperture 170 by means of the internal conduct 17.

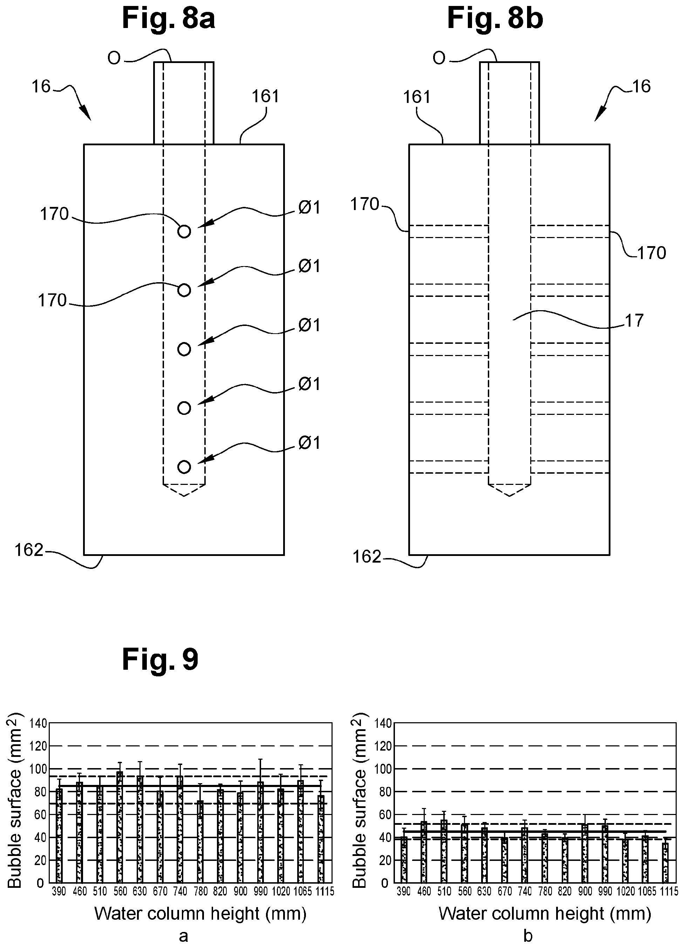

The at least radial aperture 170 presents a diameter ranging from 0.5 to 1.5mm, preferably 1mm. In the embodiment depicted on figures 6, 8a and 8b, the sparger 16 displays ten radial apertures 170, five on each side of the sparger 16. On each side, all five radial apertures 170 are aligned along the revolution axis A. Once the sparger 16 is fed with gas, each radial aperture 170 enables to produce a series of gas bubbles 26 presenting a diameter D ranging from 5 to 17mm.

The sparger 16 is therefore preferably arranged and secured in the center of the closed extremity 20 of the internal tank 14. The sparger 16 is centered with regard to the revolution axis X, thus enabling the formation of a central air column.

This makes the bioreactor 10 according to the present invention of the "bubble column" type, meaning that the agitation of the liquid is generated by the introduction of gas at the bottom of the bioreactor. Moreover, in the bioreactor 10 of the present invention being an air-lift system, the air flow of the bubble column directly influences the agitation and the type of flow in the internal tank 14 of the bioreactor 10. The higher the air of the bubble column flows, the more the liquid flow is increased. Some examples of experimental results will be discussed further below.

In order to feed the sparger 16 with gas, preferably air, the sparger 16 is put in fluidic communication with a gas injection device 28, preferably an air injection device. The gas injection device 28 is designed to generate a gas flow rate (preferably air flow rate) ranging from 0.5L/min to 10L/min, preferably 1L/min.

When the sparger is fed with gas while the internal tank 14 is filled with liquid, the internal volume 22 of the internal tank 14 is separated into a gassed and an ungassed region. The bubbles generate a vertically circulating flow between the gassed and ungassed regions, as can be seen on figure 3. The internal volume 22 is solely separated by the flowing air of the generated bubble column.

The control system 18 allows the acquisition and/or the control of several monitoring signals inside the liquid like for example pH, conductivity, dissolved oxygen. The control system comprises for example a control box like BioFlo® 120 Eppendorf or Biostat® B Sartorius. This control system 18 thus allows a precise monitoring of the growing process of the biomass.

The bioreactor 10 according to the present invention enables the implementation of a method for growing biomass in a culture medium, said culture medium being put in motion by air bubbles 26, said method comprising the following steps:

- filling the internal tank 14 with culture medium,
- inserting the biomass aimed at being grown or cultivated in the culture medium,
- turning the sparger 16 on in order to generate the bubble 26 column aimed at putting the culture medium into motion.

## EXAMPLES

The objective of the herein presented experiments was to compare two bioreactors with bubbling systems based on two sparger technologies. For that, the bubble size distribution in function of the water column height was measured by picture acquisition and image analysis. Furthermore, the effect of the bubble size on the growth of the biomass and on the production of a recombinant protein was assessed.

### Materials and methods

Bioreactor and aeration conditions

A single-use bioreactor containing 25 L of distilled water was used for the bubble size measurement acquisition. In order to obtain clear and workable pictures, the air flow rate was maintained at 1 L/min during all the experiment and for both spargers. The spargers were placed at the bottom of the bioreactor.

Bubble pictures acquisition

Pictures were taken using a camera (Canon®) mounted on a tripod with adjustable height. The distance between the camera and the bioreactor was set at 185 mm during all the experiment in order to have exactly the same capture conditions. Pictures were taken from the bottom of the bioreactor to the top with regular height steps.

Image analysis

Image analysis was done using the software ImageJ. The "Ellipse" tool of the software was used to encircle each bubble on the pictures acquired. The "Measurement Analysis" tool was then used to calculate the area of the encircled bubbles. As this data was given by the software in pixel units, a calibration was needed to know the equivalence between pixels and distance in mm. For that, a control consisting in a sphere of 50 mm diameter was used for pixels calibration. A picture of the sphere was acquired in exactly the same conditions as the bubbles and the picture analysis has allowed to establish the equivalence between millimeters and pixels. This calibration was used to calculate the bubble area in square millimeters. Bubble areas were then averaged for each height step in order to represent the distribution of the average area in function of the height of the bioreactor, for both spargers.

Biomass concentration measurement

Hairy roots of *Brassica rapa rapa* producing the protein IDUA (alpha-L-iduronidase) were cultivated in a bioreactor in standard conditions. At the end of the hairy root culture, the roots were collected, washed in protein recovery solution, rinsed with distilled water and dried at 70°C during 48 h. Dry weight (in $g_{DW}$) was then measured and reported to the culture volume (in L) to calculate the biomass concentration (in $g_{DW}$/L).

IDUA activity and productivity

To assess the amount of IDUA protein produced by the hairy root culture, the enzymatic activity of the IDUA protein was measured in the collected protein recovery solution using a fluorimetric enzymatic assay based on the fluorogenic substrate sodium 4-methylumbelliferyl-$\alpha$-L-Iduronide (4MU-I; Santa Cruz Biotechnology). The 4MUI substrate was diluted to a working solution of 400 $\mu$M 4MU-I with the reaction buffer 0.4 M sodium formate, pH 3.5. Twenty-five $\mu$L of sample to analyze were added to 25 $\mu$L of 400 $\mu$M 4MU-I substrate. The mixture was incubated at 37 °C for 30 min and 200 $\mu$L glycine carbonate buffer (pH 9.8) was added to quench the reaction. 4-Methylumbelliferone (4MU) (Sigma) was used to prepare the standard calibration curve. Fluorescence was measured using a plate reader (TECAN Infinite M1000™, Männedorf, Switzerland) with excitation at 355 nm and emission at 460 nm. IDUA activity in measured in enzymatic unit per liter of sample (U/L). IDUA productivity (in mU/$g_{DW}$/d) was then calculated by dividing the IDUA units (in U) by the dry weight ($g_{DW}$) and by the production duration (in days).

**Results and discussion**

Figure 9 - Bubble Size Measurement

Figure 9a expresses the bubble surface (in mm$^2$) in function of the water column height (in mm) when using a large bubble sparger according to the present invention. The average surface is 85.2mm$^2$ ($IC_{95}$=11.41) corresponding to an average diameter of about 5.2 mm. Figure 9b expresses the bubble surface (in mm$^2$) in function of the water column height (in mm) when using a microbubble sparger. The average surface is 42.26mm$^2$ ($IC_{95}$=6.3) corresponding to an average diameter of about 3.65 mm.
The conclusion that can be drawn from those results is that the generated bubbles are twice smaller when using a microbubble sparger than when using a large bubble sparger like in the present invention.

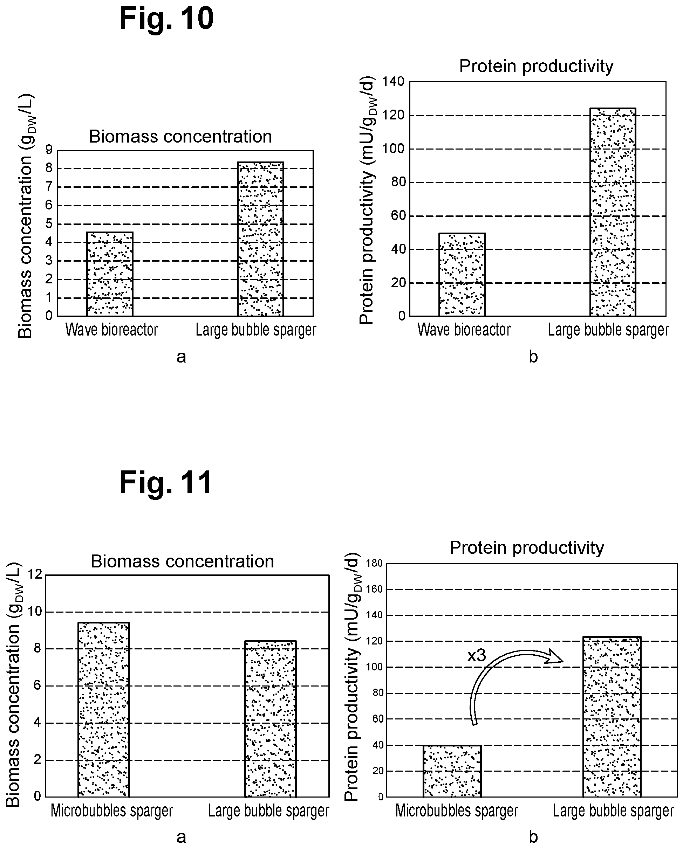

Figure 10a and 10b - Comparison with a wave bioreactor

Figure 10a shows the difference of biomass concentration in $g_{DW}$/L and the protein productivity in mU/$g_{DW}$/d obtained after 39 days of culture while using (on the left) a commercially available wave bioreactor and (on the right) a bioreactor with a large bubble sparger according to the present invention. A wave bioreactor does not oxygenate the culture medium by means of bubbles, the oxygenation is solely provided by agitation.
It appears quite clearly that the biomass concentration obtained after 39 days of culture is about two times greater when using a large bubble sparger according to the present invention than when using a commercially available wave bioreactor.
It also appears quite clearly that the amount of protein produced after 39 days of culture is about three times greater when using a large bubble sparger according to the present invention than when using a wave bioreactor.

Figure 11a and 11b - Comparison with a microbubble sparger

Figure 11a shows the biomass concentration in $g_{DW}$/L obtained after 39 days of culture while using (on the left) a bioreactor with a microbubble sparger and (on the right) a bioreactor with a large bubble sparger according to the present invention.

No significant impact on biomass production can be detected.

Figure 11b shows the difference in the amount of protein produced after 39 days of culture, expressed in mU/$g_{DW}$/d when using (on the left) a bioreactor with a microbubble sparger and (on the right) a bioreactor with a large bubble sparger according to the present invention.
Despite the similar biomass concentration observed in figure 11a, it appears quite clearly that using a bioreactor with a large bubble sparger according to the present invention leads to a protein production about three times greater than when using a microbubble bioreactor.

Conclusion

Therefore, using a bioreactor with a large bubble sparger like in the present invention allows to significantly increase the protein production by a hairy root culture, notably in comparison to the protein production by a hairy root culture in a bioreactor with a microbubble sparger or in a wave bioreactor.

**Claims**

1. Bioreactor (10) for the production of biomass, the bioreactor (10) being configured to contain at least 15 liters of liquid comprising:

    - an external support structure (12) displaying a general annular shape extending along a revolution axis (X),

- a generally cylindrical shaped internal tank (14) made of flexible material, said internal tank (14) being configured to be secured inside and supported by the external support structure (12), the internal tank (14) further displaying a closed extremity (20),

wherein the internal space (22) of the internal tank (14) is free of any internal structure, wherein the bioreactor (10) further comprises a sparger (16) located at the closed extremity (20) of the internal tank (14),wherein the sparger (16) is configured to generate bubbles (26) displaying a diameter (D) of at least 5 millimeters, the diameter D of the generated bubbles (26) ranging from 5 to 17mm, wherein the sparger (16) and the internal tank (14) are arranged in a way that the generated bubbles (26) form one single bubble column once the internal tank (14) is filled with liquid and the sparger (16) is fed with gas, said single bubble column enabling the liquid to be put in motion.

2. Bioreactor (10) according to the preceding claim, wherein the sparger (16) is arranged and secured in the center of the closed extremity (20), centered with regard to the revolution axis X.

3. Bioreactor (10) according to any one of the preceding claims, wherein the sparger (16) comprises at least one radial aperture (170) located along a revolution axis A of the sparger (16), said aperture (170) being designed to generate a column of bubbles (26).

4. Bioreactor (10) according to the preceding claim, wherein the sparger (16) comprises two alignments of at least one radial aperture (170) located along a revolution axis A of the sparger (16), the two alignments being on opposite sides of the sparger (16), said apertures (170) being designed to generate at least one column of bubbles (26).

5. Bioreactor (10) according to any one of the preceding claims, wherein the bioreactor (10) is configured to contain at least 100 liters of liquid.

6. Bioreactor (10) according to any one of the preceding claims, wherein the bioreactor (10) is configured to contain at least 350 liters of liquid.

7. Bioreactor (10) according to any one of the preceding claims, wherein the bioreactor (10) is configured to contain at least 1000 liters of liquid.

8. Bioreactor (10) according to any one of the preceding claims, wherein the internal tank (14) is disposable.

9. Bioreactor (10) according to any one of the preceding claims, wherein it further comprises a control system (18).

10. Bioreactor (10) according to any one of the preceding claims, wherein the closed extremity (20) displays a conical shape centered around the revolution axis (X).

11. Bioreactor (10) according to the precedent claim, wherein the sparger (16) is designed to cooperate with the conical shape of the closed extremity (20).

12. Bioreactor (10) according to any one of the precedent claim, wherein the bioreactor (10) is an air-lift system in which the liquid is solely put in motion by the generated air bubbles (16).

13. System for the production of biomass, said system comprising a bioreactor (10) according to any one of the preceding claims and a gas injection device, the gas injection device being put in fluidic communication with the sparger (16).

14. Method for growing biomass in a culture medium, said culture medium being put in motion by a bioreactor (10) according to any one of claims 1 to 12 said method
comprising the following steps:

- filling the internal tank (14) with culture medium,
- inserting the biomass aimed at being grown in the culture medium,
- feeding the sparger (16) with gas in order to generate the bubble column aimed at putting the culture medium into motion.

**Patentansprüche**

1. Bioreaktor (10) zur Produktion von Biomasse, wobei der Bioreaktor (10) so eingerichtet ist, dass er mindestens 15 Liter Flüssigkeit enthält, umfassend:

- eine äußere Stützstruktur (12), die eine allgemeine Ringform aufweist und sich entlang einer Umdrehungsachse (X) erstreckt,
- einen im Allgemeinen zylindrisch geformten Innentank (14) aus flexiblem Material, wobei der Innentank (14) so eingerichtet ist, dass er innerhalb der äußeren Stützstruktur (12) gesichert ist und von dieser getragen wird, wobei der Innentank (14) ferner ein geschlossenes Ende (20) aufweist,

wobei der Innenraum (22) des Innentanks (14) frei von jeglicher innerer Struktur ist, wobei der Bioreaktor (10) einen Sparger (16) umfasst, der sich am geschlossenen Ende (20) des Innentanks (14) befindet, **wobei** der Sparger (16) so eingerichtet ist, dass er Blasen (26) erzeugt, die einen Durchmesser (D) von mindestens 5 Millimetern aufweisen, wobei der Durchmesser D der erzeugten Blasen (26) im Bereich von 5 bis 17 mm liegt, **wobei** der Sparger (16) und der Innentank (14) derart angeordnet sind, dass die erzeugten Blasen (26) eine einzige Blasensäule bilden, sobald der Innentank (14) mit Flüssigkeit gefüllt ist und der Sparger (16) mit Gas versorgt wird, wobei diese einzelne Blasensäule es ermöglicht, die Flüssigkeit in Bewegung zu versetzen.

2. Bioreaktor (10) nach dem vorhergehenden Anspruch, wobei der Sparger (16) in der Mitte des geschlossenen Endes (20), bezogen auf die Umdrehungsachse X zentriert, angeordnet und gesichert ist.

3. Bioreaktor (10) nach einem der vorhergehenden Ansprüche, wobei der Sparger (16) mindestens eine radiale Öffnung (170) umfasst, die sich entlang einer Umdrehungsachse A des Spargers (16) befindet, wobei die Öffnung (170) so ausgebildet ist, dass sie eine Säule aus Blasen (26) erzeugt.

4. Bioreaktor (10) nach dem vorhergehenden Anspruch, wobei der Sparger (16) zwei Ausrichtungen von mindestens einer radialen Öffnung (170) umfasst, die sich entlang einer Umdrehungsachse A des Spargers (16) befinden, wobei sich die beiden Ausrichtungen auf gegenüberliegenden Seiten des Spargers (16) befinden, wobei die Öffnungen (170) so ausgebildet sind, dass sie mindestens eine Säule aus Blasen (26) erzeugen.

5. Bioreaktor (10) nach einem der vorhergehenden Ansprüche, wobei der Bioreaktor (10) so eingerichtet ist, dass er mindestens 100 Liter Flüssigkeit enthält.

6. Bioreaktor (10) nach einem der vorhergehenden Ansprüche, wobei der Bioreaktor (10) so eingerichtet ist, dass er mindestens 350 Liter Flüssigkeit enthält.

7. Bioreaktor (10) nach einem der vorhergehenden Ansprüche, wobei der Bioreaktor (10) so eingerichtet ist, dass er mindestens 1000 Liter Flüssigkeit enthält.

8. Bioreaktor (10) nach einem der vorhergehenden Ansprüche, wobei der Innentank (14) ein Einwegartikel ist.

9. Bioreaktor (10) nach einem der vorhergehenden Ansprüche, wobei er ferner ein Steuersystem (18) umfasst.

10. Bioreaktor (10) nach einem der vorhergehenden Ansprüche, wobei das geschlossene Ende (20) eine konische Form aufweist, die um die Umdrehungsachse (X) zentriert ist.

11. Bioreaktor (10) nach dem vorhergehenden Anspruch, wobei der Sparger (16) so ausgebildet ist, dass es sich der konischen Form des geschlossenen Endes (20) anpasst.

12. Bioreaktor (10) nach einem der vorhergehenden Ansprüche, wobei der Bioreaktor (10) ein Airlift-System ist, bei dem die Flüssigkeit ausschließlich durch die erzeugten Luftblasen (16) in Bewegung versetzt wird.

13. System zur Erzeugung von Biomasse, wobei das System einen Bioreaktor (10) nach einem der vorhergehenden Ansprüche und eine Gaseinspritzvorrichtung umfasst, wobei die Gaseinspritzvorrichtung in fluidische Kommunikation mit dem Sparger (16) gebracht wird.

14. Verfahren zum Anbauen von Biomasse in einem Kulturmedium, wobei das Kulturmedium von einem Bioreaktor (10) nach einem der Ansprüche 1 bis 12 in Bewegung versetzt wird, wobei das Verfahren die folgenden Schritte umfasst:

- Befüllen des Innentanks (14) mit Kulturmedium,
- Einbringen der Biomasse, die darauf abzielt, im Kulturmedium angebaut zu werden,
- Speisen des Spargers (16) mit Gas, um die Blasensäule zu erzeugen, die darauf abzielt, das Kulturmedium in Bewegung zu versetzen.

**Revendications**

1. Bioréacteur (10) destiné à la production de biomasse, ledit bioréacteur (10) étant conçu pour contenir au moins 15 litres de liquide, comprenant :

- une structure de support externe (12) présentant une forme générale annulaire s'étendant le long d'un axe de révolution (X),
- un réservoir interne (14) de forme généralement cylindrique, réalisé en un matériau souple, ledit réservoir interne (14) étant configuré pour être fixé à l'intérieur de la structure de support externe (12) et supporté par celle-ci, le réservoir interne (14) présentant en outre une extrémité fermée (20),

dans lequel l'espace interne (22) de la cuve interne (14) est dépourvu de toute structure interne, le bioréacteur (10) comprenant en outre un diffuseur (16) situé à l'extrémité fermée (20) de la cuve interne (14), dans lequel le diffuseur (16) est configuré pour générer des bulles (26) présentant un diamètre (D) d'au moins 5 millimètres, le diamètre D des bulles générées (26) étant compris entre 5 et 17 mm, dans lequel le diffuseur (16) et le réservoir interne (14) sont agencés de telle sorte que les bulles générées (26) forment une seule colonne de bulles une fois que le réservoir interne (14) est rempli de liquide et que le diffuseur (16) est alimenté en gaz, ladite colonne unique de bulles permettant de mettre le liquide en mouvement.

2. Bioréacteur (10) selon la revendication précédente, dans lequel le diffuseur (16) est disposé et fixé au centre de l'extrémité fermée (20), en étant centré par rapport à l'axe de révolution X.

3. Bioréacteur (10) selon l'une quelconque des revendications précédentes, dans lequel le diffuseur (16) comprend au moins une ouverture radiale (170) située le long d'un axe de révolution A du diffuseur (16), ladite ouverture (170) étant conçue pour générer une colonne de bulles (26).

4. Bioréacteur (10) selon la revendication précédente, dans lequel le diffuseur (16) comprend deux rangées d'au moins une ouverture radiale (170) situées le long d'un axe de révolution A du diffuseur (16), les deux rangées se trouvant sur des côtés opposés du diffuseur (16), lesdites ouvertures (170) étant conçues pour générer au moins une colonne de bulles (26).

5. Bioréacteur (10) selon l'une quelconque des revendications précédentes, dans lequel le bioréacteur (10) est configuré pour contenir au moins 100 litres de liquide.

6. Bioréacteur (10) selon l'une quelconque des revendications précédentes, dans lequel le bioréacteur (10) est conçu pour contenir au moins 350 litres de liquide.

7. Bioréacteur (10) selon l'une quelconque des revendications précédentes, dans lequel le bioréacteur (10) est conçu pour contenir au moins 1 000 litres de liquide.

8. Bioréacteur (10) selon l'une quelconque des revendications précédentes, dans lequel le réservoir interne (14) est jetable.

9. Bioréacteur (10) selon l'une quelconque des revendications précédentes, **caractérisé en ce qu'**il comprend en outre un système de commande (18).

10. Bioréacteur (10) selon l'une quelconque des revendications précédentes, dans lequel l'extrémité fermée (20) présente une forme conique centrée autour de l'axe de révolution (X).

11. Bioréacteur (10) selon la revendication précédente, dans lequel le diffuseur (16) est conçu pour coopérer avec la forme conique de l'extrémité fermée (20).

12. Bioréacteur (10) selon l'une quelconque des revendications précédentes, dans lequel le bioréacteur (10) est un système à ascension d'air dans lequel le liquide est mis en mouvement uniquement par les bulles d'air générées (16).

13. Système pour la production de biomasse, ledit système comprenant un bioréacteur (10) selon l'une quelconque des revendications précédentes et un dispositif d'injection de gaz, le dispositif d'injection de gaz étant mis en communication fluidique avec le diffuseur (16).

14. Procédé de culture de biomasse dans un milieu de culture, ledit milieu de culture étant mis en mouvement par un bioréacteur (10) selon l'une quelconque des revendications 1 à 12, ledit procédé comprenant les étapes suivantes :

   - remplir la cuve interne (14) avec le milieu de culture,
   - introduire la biomasse destinée à être cultivée dans le milieu de culture,
   - alimenter le diffuseur (16) en gaz afin de générer la colonne de bulles destinée à mettre le milieu de culture en mouvement.

# Fig. 1

Probe
Filter
Thermal resistance
Pump
Measurement chamber

11

10

10B

X

-130-

131

14

12

-22-

132

pH

Cond

O2

-18-

28

16

20

10A

# Fig. 2

14

10

12

16

# Fig. 3

14

26
26
26
26

# Fig. 4

# Fig. 5

# Fig. 6

# Fig. 7

# Fig. 8a

# Fig. 8b

# Fig. 9

a

b

# Fig. 10

Biomass concentration

Biomass concentration (g_DW/L)

Wave bioreactor — Large bubble sparger

a

Protein productivity

Protein productivity (mU/g_DW/d)

Wave bioreactor — Large bubble sparger

b

# Fig. 11

Biomass concentration

Biomass concentration (g_DW/L)

Microbubbles sparger — Large bubble sparger

a

Protein productivity

Protein productivity (mU/g_DW/d)

x3

Microbubbles sparger — Large bubble sparger

b

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2008135991 A2

- US 2007037279 A